# EUROPEAN PATENT SPECIFICATION

(11) **EP 3 706 811 B1**
(45) Date of publication and mention of the grant of the patent: **29.11.2023**
(21) Application number: 18795647.9
(22) Date of filing: 31.10.2018
(51) Int. Cl.: A61L 2/26, B29D 11/00

(54) **STERILISATION RACK AND STERILISATION METHOD USING SUCH RACKS**
STERILISATIONSREGAL UND STERILISATIONSVERFAHREN MIT SOLCHEN REGALEN
ÉTAGÈRE DE STÉRILISATION ET PROCÉDÉ DE STÉRILISATION UTILISANT DE TELLES ÉTAGÈRES

(30) Priority: 09.11.2017 EP 17200735
(43) Date of publication of application: 16.09.2020
(73) Proprietor: SAV-IOL SA, 2000 Neuchatel (CH)
(72) Inventor: JACOT, Julien Vincent, 2074 Marin-Epagnier (CH); PASCARELLA, Alexandre, 2013 Colombier (CH)
(74) Representative: Omnis-IP
(86) International application number: PCT/EP2018/079867
(87) International publication number: WO 2019/091849

(56) References cited:
- EP-A1- 0 283 928
- WO-A1-2016/142331
- WO-A2-03/074093
- JP-A- H1 111 436
- US-A1- 2015 320 503

## Description

### TECHNICAL FIELD

The present invention concerns a sterilisation rack for holding containers containing articles to be sterilised. The invention further concerns a method for sterilising objects placed in a container.

### BACKGROUND ART

Currently, many products, in particular products for medical use, must be sterilised before being used. Some of these products are placed in a liquid-containing plastic container so that they can be kept under optimal conditions prior to their use. These containers generally have a lid, typically a peelable lid, so that on the one hand, the container is securely sealed when the lid is in place and, on the other hand, this lid can be easily removed in order to access the product. Ophthalmic lenses, particularly intraocular lenses, are often packaged in such containers.

During sterilisation, these containers are placed in a medium which is heated to a certain temperature. The containers are held for a time at this temperature or, more generally, undergo a sterilisation cycle during which the temperature is increased relative to the ambient temperature, before being cooled to this ambient temperature. Due to these warming and cooling phases and to the presence of liquid in the container and furthermore to the fact that the container is sealed in a leak-tight manner, the pressure inside the container increases. This increase in pressure can cause a deformation of the container, both at the level of the lid and of the bottom of the container.

Depending on the use of the product to be sterilised, such deformations are not acceptable. Indeed, these deformations can lead to a risk of separation of the lid, calling into question the quality of the sealing and therefore, the package integrity. Internal pressure can lead directly to leakages during the sterilisation process, which of course invalidates any sterile conditions. Depending on the product, it is not possible to re-package the product in a new container and to undergo a new sterilisation phase. The separation of the lid therefore means that the product must be discarded.

The deformations make the stacking of the containers more complicated during their storage, transport and/or distribution, which can lead to the containers falling. Moreover, these deformations may give the impression that the sterilisation is poorly controlled.

Solutions have been developed to solve some of these problems. One solution is to sterilise products and containers in an overpressure autoclave. In such autoclaves, the pressure is greatly increased with respect to atmospheric pressure. This overpressure in the autoclave is such that during the sterilisation cycle, the pressure inside the autoclave, outside the container, is approximately equal to the maximum pressure reached inside the container. As this pressure can be particularly high, it is necessary to use an autoclave which is capable of generating the required pressure and which is able to resist to such a pressure. Such systems are relatively expensive and are therefore not accessible to all persons for all applications.

For example, International Patent Application Publication Number WO 03/074093 A2 discloses a raised temperature sterilisation process in which a plurality of soft contact lens packages containing soft contact lenses to be sterilised are automatically transported through an in-line apparatus in novel sterilisation trays. The sterilisation trays are for carrying nested stacks of blister pack arrays carrying ophthalmic lenses for input to a steriliser machine. The sterilisation trays comprise external walls and internal walls dividing the tray to allow them to hold a plurality of ophthalmic lens packages in the most efficient orientation for sterilising. According to the publication, each tray has a capacity for 660 packages of interleaved pairs of three blister packs. Since throughput is of primary concern, the trays are generally fully loaded so that simultaneous sterilisation of 5,940 packages may take place. If the full capacity described above is not required, then spaces for the blister packages may simply be left empty. The blister packages contain an aqueous solution. As it is known that a heated environment causes the pressure in such packages to rise, the sterlisation machine described in the publication includes a system of valves to maintain an over-pressure in the sterilisation chamber. This guarantees the integrity of the blister packages during the sterilisation process.

The publication WO 03/074093 describes a sterilization apparatus and method for ophthalmic lenses packages an in-line apparatus in order to sterilize these ophthalmic lenses. The apparatus comprises several trays in which ophthalmic lenses packages are placed prior to being introduced in a sterilization apparatus. Each tray comprises divider walls, two adjacent divider walls defining a cavity in which one lens package can be placed.

Space is provided between the divider walls and the package and the divider walls cannot be moved in order to apply a counter-pressure to the packages when the internal pressure raises. No mechanical counter-pressure is applied on the packages and the sterilization must be caried out in an overpressure autoclave.

The publication JP H11 11436 describes a device for holding containers in an aligned state, said containers containing devices or products that must be steam-sterilized. In said device, the containers are filed with ozone and are immersed in ozone water, in order to avoid the drawbacks encountered by heating the container in a steam sterilization apparatus.

As the container is not submitted to an increase of the internal pressure, no counter-pressure mechanism is required.

### DISCLOSURE OF INVENTION

The present invention provides for a device to allow the sterilisation of products contained in deformable containers while avoiding the deformation of the containers in a conventional autoclave environment, whose raised temperature generally contributes to a build-up of pressure within the containers due to an increase in volume of fluid within the containers. Consequently, the sterilisation can be carried out in a conventional autoclave. This makes it possible to use relatively inexpensive material, which allows for reliable, simple and cost-effective large-scale sterilisation for a large number of applications.

Through the use of the device disclosed herein, the containers do not become deformed, which makes it possible to ensure that the lid does not become detached and that the contents therefore remain sterile. The fact that the containers do not become deformed also makes it possible to stack the containers in a way that avoids the risk of falling, especially during storage and transport of the containers.

An object of the invention is achieved by a rack for holding one or more products to be sterilised in a raised temperature environment, each of the products being held in a container which is deformable due to an increased pressure within the container when placed in said environment, the rack comprising:
an elongate base having a first end and a second end at opposite ends along the base;
a buffer plate located at a first position along the base;
a compression unit comprising a pressure plate, the compression unit being located at a second position towards one of the ends of the base; and
one or more container holders, located between the buffer plate and the pressure plate, each container holder being adapted to retain its respective container;
the compression unit suitable to apply pressure to said container holders;
characterised in that the buffer is placed on the base and said buffer plate comprises means so that the first position where the buffer plate is located may be selected from a plurality of alternative first positions along the base and;
the compression unit comprises a threaded bore through which a threaded spindle of the pressure plate is adapted to engage and is actionable to adjust the position of the pressure plate in a longitudinal direction with respect to the location of the compression unit such that contact is made between the pressure plate and each successive container holder up to the buffer plate.

An object of the invention is also achieved by a method for preparing one or more products for sterilisation in a raised temperature environment, the products each being held in a container which is deformable due to an increased pressure within the container when placed in said environment, the method comprising:
retaining each of the containers in a respective container holder such that deformable surfaces of the container are supported by the container holder;
assembling one or more of the containers with their container holders on a sterilisation rack, the sterilisation rack having:
   an elongate base having a first end and a second end at opposite ends along the base;
   a buffer plate located at a first position along the base; and
   a compression unit comprising a pressure plate, the compression unit comprising a threaded bore through which a threaded spindle of the pressure plate is adapted to engage and is actionable to adjust the position of the pressure plate in a longitudinal direction with respect to the location of the compression unit the compression unit being located at a second position towards one of the ends of the base;
adjusting a position of the buffer plate along the base so that said containers fit between the buffer plate and the pressure plate;
adjusting a position of the pressure plate by actioning said threaded spindle such that contact is made between the pressure plate and each successive container holder up to the buffer plate.

The sterilisation rack according to the invention is intended to be placed in an autoclave, the containers to be sterilised being arranged in the rack. This rack holds the containers to be sterilised and provides counter pressure which counteracts the pressure generated inside the containers by the increase of the temperature during sterilisation. The shape of the sterilisation rack can easily be adapted to accommodate containers of different shapes and sizes. The rack can be easily adapted to accommodate different numbers of containers. In addition, it is possible, in the same autoclave, to place several racks, so as to sterilise a large number of containers. The rack according to the invention is relatively inexpensive so that it can be used in a large number of applications and in particular in applications for which it would not be possible to accept a high cost of sterilisation.

The sterilisation method according to the invention is relatively easy to implement and does not require a particular autoclave. This method can therefore be implemented in a large number of applications.

### BRIEF DESCRIPTION OF DRAWINGS

The present invention and its advantages will be better understood with reference to the enclosed drawings and to the detailed description of a specific embodiment, in which:

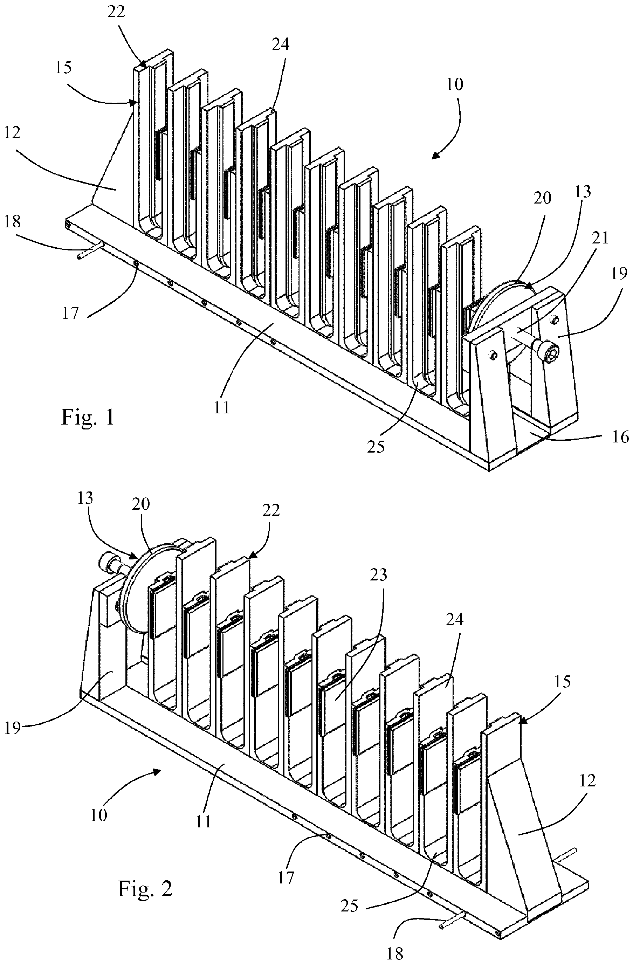

- Fig. 1 is a perspective view of a sterilisation rack according to an embodiment of the invention, as seen from a first side of the rack;
- Fig. 2 is a perspective view similar to that of Fig. 1, as seen from a second side of the rack;

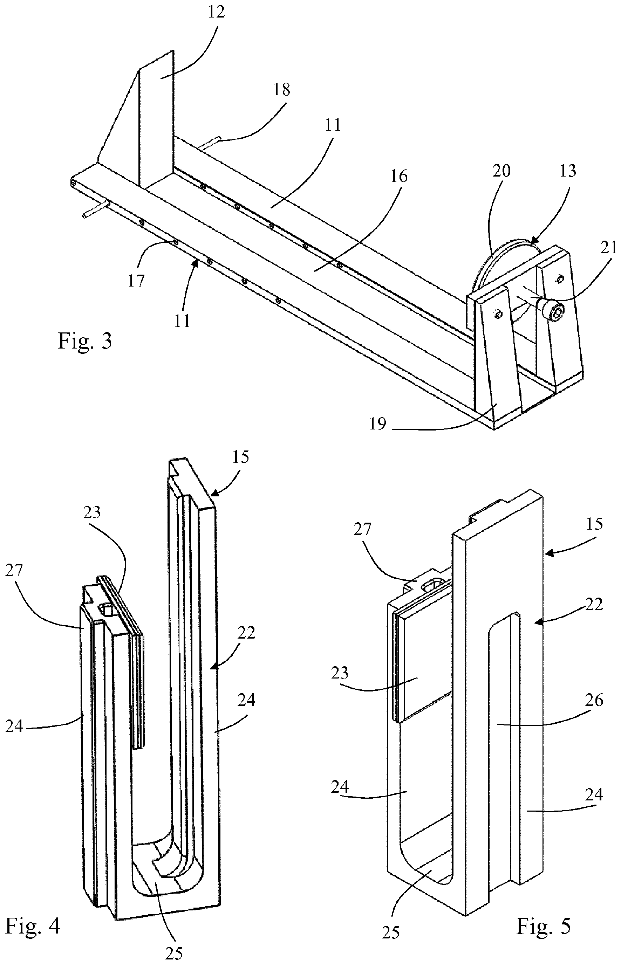

- Fig. 3 is a perspective view of the rack of Figs. 1 and 2 without the container holders;
- Fig. 4 shows a container holder comprised within a rack according to an embodiment of the invention, as viewed from a first direction;
- Fig. 5 shows the container holder of Fig. 4, viewed from a second direction;

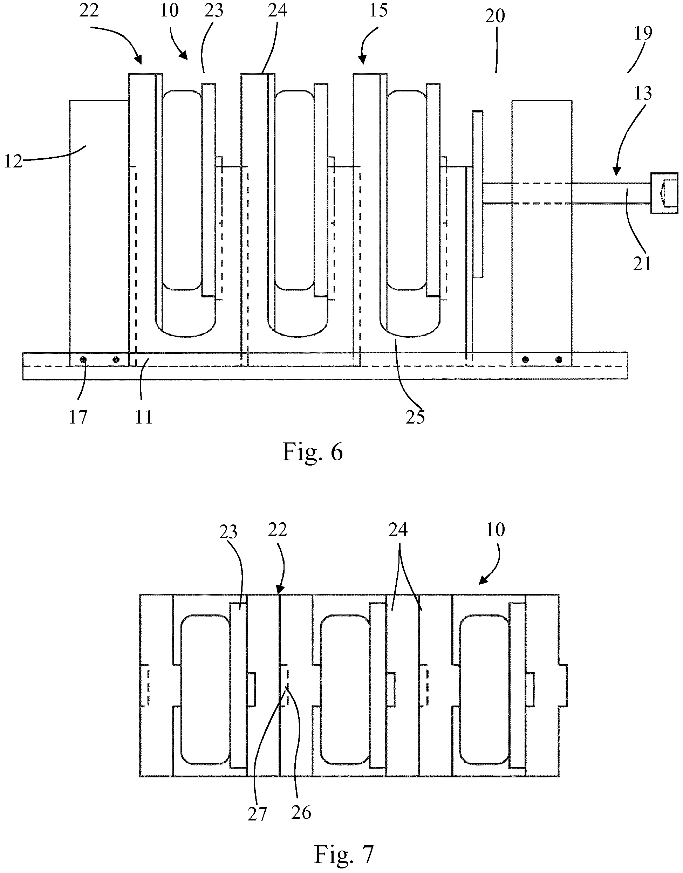

- Fig. 6 is a lateral view of a sterilisation rack, in which containers to be sterilised have been placed; and
- Fig. 7 is a top view of the rack of Fig. 6.

### MODES FOR CARRYING OUT THE INVENTION

With reference to the figures, the sterilisation rack 10 according to the invention essentially comprises a base 11, a buffer plate 12, a compression unit 13, and a plurality of container holders 15.

The base 11 of the rack is of substantially elongated form, having a first end and a second end opposite from each other in the longitudinal direction of the base, and is provided with a longitudinal groove 16. The base 11 also comprises transversal bores 17 passing through the longitudinal groove 16 each suitable for accepting the passage of a transversal rod 18.

The buffer plate 12 is designed to be placed on the base 11 and its position may be selected from a plurality of alternative positions along the base. This buffer plate comprises a buffer plate face substantially perpendicular to the elongate base and an engaging face substantially coplanar with the base of the rack. A section of this buffer plate in a longitudinal direction with respect to the base of the rack may have the shape of a rectangle, a right-angled triangle or a set square for example. In the embodiment illustrated, the width of the buffer plate 12 is substantially equal to or slightly less than the width of the longitudinal groove 16 of the base plate so that the engaging face of the buffer plate can be placed into and slid along the longitudinal groove.

The buffer plate 12 further comprises at least one through-bore, arranged to be placed in front of at least one transversal bore 17 of the base. The buffer plate can be positioned at different locations along the base in its longitudinal direction and held in position by means of the rod 18 passing through two transversal bores 17 of the base and through the through-bore of the buffer plate.

It is clear that other means for maintaining the buffer plate 12 may be considered, such as for example the use of screws fixed under the base plate in particular.

The compression unit 13 comprises at least one arm 19 which may be formed integrally with the base 11 or can be made integral therewith. The compression unit can also be fixed to the base or it can be made integral with the base in a chosen position, in a manner similar to the buffer plate 12. According to the embodiment illustrated, the arm 19 is secured to the base and is fixed in a predetermined position, for example by means of screws. According to a variant, the arm could have a shape similar to that of the buffer plate and be made integral with the base by means of a rod passing through a transversal bore of the base and a through bore of the arm.

The compression unit 13 further comprises a pressure plate 20 arranged in a plane perpendicular to the longitudinal axis of the base. This pressure plate 20 is movable along this longitudinal axis, that is to say in a direction parallel to the longitudinal groove 16 of the base. To this end, the pressure plate 20 has a threaded spindle 21 allowing it to be mounted on the compression unit by threading the spindle through a threaded bore of the arm of the compression unit. The rotation of the threaded spindle 21 has the effect of moving the pressure plate 20 forward or backward through the arm of the compression unit while the compression unit remains fixed to the base. This threaded spindle 21 can be actuated by an actuating element which can take various forms such as a crank, a socket head screw or a thumb screw, for example.

The sterilisation rack 10 according to the invention comprises a plurality of container holders 15, in principle as many as there are containers to be placed in this rack. In the case where it is intended for only one container to be placed in the rack there will be only one container holder. Each container holder 15 is in principle intended to receive one container. A plurality of these container holders 15 is intended to be placed in the rack between the buffer plate 12 and the pressure plate of the compression unit 13.

A container holder 15 according to an embodiment of the invention is formed of a clamp 22 and a counter-pressure plate 23. The clamp 22 comprises two holding legs 24, which are parallel to each other and intended to be positioned perpendicularly to the plane of the base. These two holding legs 24 are interconnected by a guiding leg 25 that is substantially perpendicular to the two holding legs 24. This guiding leg 25 has a profile complementary to the profile of the base and is designed to allow longitudinal sliding of the guiding leg 25 of the container holder 15 in the base 11, along the longitudinal groove 16.

According to an advantageous embodiment, an external face of a holding leg 24 of the clamp 22 comprises a profile 26 provided with a recess while the external face of the other holding leg 24 comprises a complementary profile 27. This makes it possible to position several clamps 22 against each other and to make them integral with one another.

The internal face of at least one of the holding legs comprises a housing arranged to cooperate with a complementary protuberance formed in the counter-pressure plate 23. This makes it possible to make this counter-pressure plate integral with the clamp 22 of the container holder 15.

Depending on the shape of the housing and the protuberance, it is possible to slide the counter-pressure plate 23 so as to adapt it to the position of the container. It is also possible to use a different counter-pressure plate according to the shape of the container, without changing the shape of the clamps.

When containers and their contents are to be sterilised, a container is placed in a container holder 15, the counter-pressure plate 23 being placed in front of the container lid. Several container holders 15 are aligned with one another, the profiles 26, 27 of the holding legs 24 cooperating in such a way as to fix the clamps 22 to a certain extent with one another. The set of container holders is arranged on the base. The buffer plate 12 is positioned at one of the alternative positions along the base so as to leave a given space between the buffer plate and the compression unit 13. The given space is preferably wide enough to receive the number of container holders for sterilising the desired number of products.

Once the desired number of products are loaded into the rack in their respective containers, the compression unit 13 is then actuated so as to apply pressure to the container holders 15. The rack can then be placed in an autoclave and undergo a sterilisation cycle therein. It follows from the design of the rack, where successive container holders are preferably interlocked, that the container holders become sandwiched between the pressure plate and the buffer plate. The position of the buffer plate is adjustable along the base of the rack to account for any number of container holders such that the actuation of the compression unit causes contact to be made between the pressure plate and each successive container holder up to the buffer plate. Consequently, the counter-pressure plates of each of the container holders provide counter-pressure against the expanding containers as they are subjected to the raised temperature environment of the sterilisation process.

By properly adjusting the position of the buffer plate and adjusting the pressure plate so that the container holders are held sandwiched together, with no empty spaces between the pressure plate and the buffer plate, this ensures an even distribution of counter-pressure between the containers, or blister packs, as they are sterilised since each of the containers generally holds the same contents, a lens and a given volume of fluid. Consequently, there is an auto-equilibration of counter-pressure throughout the system.

According to a particular embodiment of the present invention, the clamp does not comprise a counter-pressure plate. Instead, the function of the counter-pressure plate in the embodiment described above is realised by the internal face of the holding leg 24 of the clamp 22. In this case the container holder 15 is formed simply of the clamp 22, the container holder and the clamp being one and the same. In this particular embodiment, the internal face of the clamp does not comprise a housing as described above. The internal face serves to provide the counter-pressure against the container to prevent it from deforming as described above.

The container holders can be said to be adapted to retain their respective containers because the container should fit snugly within the clamp so that when the pressure plate is adjusted for the sterilising process to begin the clamps or the counter-pressure plates will be able to properly apply the counter-pressure to ensure that the increased pressure within the containers does not exert excessive pressure on the containers causing them to become deformed.

Having contact between the pressure plate and each successive container holder up to the buffer plate means that no spaces are left between the buffer plate and the pressure plate. All positions between the plates are occupied by a container holder. All container holders in a series of container holders along the rack therefore touch each other and the end container holders touch the buffer plate and the pressure plate, respectively. When containers are placed in their respective container holders, which are preferably configured to provide a snug fit, without leaving any container holders empty, this ensures that when any pressure builds up in the containers, there will be a counter-pressure applied by the container holders or their counter-pressure plates. The counter-pressure is evenly spread along the holders of the entire rack because the pressure level in each container is substantially equal due to each container holding the same volume of liquid.

The present invention is advantageous in particular because it allows the containers to be sterilised without deforming the lids, which are held in position by the counter-pressure plate. This may be achieved in a conventional autoclave.

The sterilisation rack can very easily be adapted to receive from one to several containers. The placement of the containers is very fast and very simple. In addition, the elements that form the rack, and in particular the container holder and the counter-pressure plate, can easily be changed so as to adapt to the shape of the container whose contents are to be sterilised.

## Claims

1. A rack (10) for holding one or more products to be sterilised in a raised temperature environment, each of the products being held in a container which is deformable due to an increased pressure within the container when placed in said environment, the rack (10) comprising:
an elongate base (11) having a first end and a second end at opposite ends along the base;
a buffer plate (12) located at a first position along the base (11);
a compression unit (13) comprising a pressure plate (20), the compression unit (13) being located at a second position towards one of the ends of the base (11); and
one or more container holders (15), located between the buffer plate (12) and the pressure plate (20), each container holder (15) being adapted to retain its respective container;
the compression unit (13) suitable to apply pressure to said container holders (15);
**characterised in that** the buffer plate (12) is placed on the base (11) and said buffer plate (12) comprises means so that the first position where the buffer plate (12) is located may be selected from a plurality of alternative first positions along the base (11) and;
the compression unit (13) comprises a threaded bore through which a threaded spindle (21) of the pressure plate (20) is adapted to engage and is actionable to adjust the position of the pressure plate in a longitudinal direction with respect to the location of the compression unit (13) such that contact is made between the pressure plate (20) and each successive container holder (15) up to the buffer plate (12).

2. The rack according to claim 1, wherein the container holder (15) comprises a clamp (22) comprising two holding legs (24) for retaining its respective container.

3. The rack according to claim 2, wherein the container holder (15) further comprises a counter-pressure plate (23) on one of the holding legs (24) for retaining its respective container.

4. The rack according to claim 3, wherein the counter-pressure plate (23) comprises a protuberance cooperating with a profile (27) of the holding leg (24) in order to adjust the position of said counter-pressure plate (23) to cause the counter-pressure plate to provide counter-pressure to a desired part of the container.

5. The rack according to any of the preceding claims, wherein the position of the compression unit (13) along the length of the base (11) is adjustable.

6. The rack according to any of the preceding claims, wherein the base (11) comprises a longitudinal groove (16), said holding legs (24) of the container holders (15) being linked together by a guiding leg (25), said guiding leg (25) being slidable along the longitudinal groove (16).

7. The rack according to any of claims 2 to 6, wherein one of the holding legs (24) of the container holders (15) have a profile (26) on their external faces and the remaining holding legs (24) of the container holders (15) have a complementary profile (27) on their external faces such that adjacent container holders interlock with each other when assembled on the base (11).

8. A method for preparing one or more products for sterilisation in a raised temperature environment, the products each being held in a container which is deformable due to an increased pressure within the container when placed in said environment, the method comprising:
retaining each of the containers in a respective container holder (15) such that deformable surfaces of the container are supported by the container holder;
assembling one or more of the containers with their container holders (15) on a sterilisation rack (10), the sterilisation rack (10) having:
an elongate base (11) having a first end and a second end at opposite ends along the base;
a buffer plate (12) located at a first position along the base (11); and
a compression unit (13) comprising a pressure plate (20), the compression unit (13) comprising a threaded bore through which a threaded spindle (21) of the pressure plate (20) is adapted to engage and is actionable to adjust the position of the pressure plate in a longitudinal direction with respect to the location of the compression unit (13) the compression unit being located at a second position towards one of the ends of the base (11);
adjusting a position of the buffer plate (12) along the base (11) so that said containers fit between the buffer plate (12) and the pressure plate (20);
adjusting a position of the pressure plate (20) by actioning said threaded spindle (21) such that contact is made between the pressure plate (20) and each successive container holder (15) up to the buffer plate (12).

## Patentansprüche

1. Gestell (10) zum Halten eines oder mehrerer zu sterilisierender Produkte in einer Umgebung mit erhöhter Temperatur, wobei jedes der Produkte in einem Behälter gehalten wird, der aufgrund eines erhöhten Drucks im Behälter verformbar ist, wenn er in der genannten Umgebung platziert wird, wobei das Gestell (10) Folgendes umfasst:
eine längliche Basis (11) mit einem ersten Ende und einem zweiten Ende an gegenüberliegenden Enden entlang der Basis;
eine Pufferplatte (12), die an einer ersten Position entlang der Basis (11) angeordnet ist;
eine Kompressionseinheit (13), die eine Druckplatte (20) umfasst, wobei die Kompressionseinheit (13) an einer zweiten Position in Richtung eines der Enden der Basis (11) angeordnet ist; und
einen oder mehrere Behälterhalter (15), die zwischen der Pufferplatte (12) und der Druckplatte (20) angeordnet sind, wobei jeder Behälterhalter (15) seinen jeweiligen Behälter halten kann;
wobei die Kompressionseinheit (13) geeignet ist, um Druck auf die Behälterhalter (15) auszuüben;
**dadurch gekennzeichnet, dass** die Pufferplatte (12) auf der Basis (11) platziert ist und die Pufferplatte (12) Mittel umfasst, so dass die erste Position, an der sich die Pufferplatte (12) befindet, aus einer Vielzahl alternativer erster Positionen entlang der Basis (11) ausgewählt werden kann, und;
die Kompressionseinheit (13) eine Gewindebohrung umfasst, durch die eine Gewindespindel (21) der Druckplatte (20) eingreifen kann und betätigbar ist, um die Position der Druckplatte in Längsrichtung in Bezug auf die Position der Druckplatte der Kompressionseinheit (13) so einzustellen, dass zwischen der Druckplatte (20) und dem jeweils nachfolgenden Behälterhalter (15) bis zur Pufferplatte (12) ein Kontakt hergestellt wird.

2. Gestell nach Anspruch 1, wobei der Behälterhalter (15) eine Klemme (22) mit zwei Halteschenkeln (24) zum Halten seines jeweiligen Behälters umfasst.

3. Gestell nach Anspruch 2, wobei der Behälterhalter (15) ferner eine Gegendruckplatte (23) an einem der Halteschenkel (24) zum Halten seines jeweiligen Behälters umfasst.

4. Gestell nach Anspruch 3, wobei die Gegendruckplatte (23) einen Vorsprung umfasst, der mit einem Profil (27) des Halteschenkels (24) zusammenwirkt, um die Position der Gegendruckplatte (23) zu verstellen, um zu bewirken, dass die Gegendruckplatte einen Gegendruck auf einen gewünschten Teil des Behälters ausübt.

5. Gestell nach einem der vorhergehenden Ansprüche, wobei die Position der Kompressionseinheit (13) entlang der Länge der Basis (11) einstellbar ist.

6. Gestell nach einem der vorhergehenden Ansprüche, wobei die Basis (11) eine Längsnut (16) umfasst, wobei die Halteschenkel (24) der Behälterhalter (15) durch einen Führungsschenkel (25) miteinander verbunden sind, wobei der Führungsschenkel (25) entlang der Längsnut (16) verschiebbar ist.

7. Gestell nach einem der Ansprüche 2 bis 6, wobei einer der Halteschenkel (24) der Behälterhalter (15) an seiner Außenseite ein Profil (26) und die übrigen Halteschenkel (24) der Behälterhalter (15) an ihren Außenflächen ein komplementäres Profil (27) aufweisen, so dass benachbarte Behälterhalter ineinandergreifen, wenn sie auf der Basis (11) zusammengebaut werden.

8. Verfahren zum Vorbereiten eines oder mehrerer Produkte für die Sterilisation in einer Umgebung mit erhöhter Temperatur, wobei die Produkte jeweils in einem Behälter gehalten werden, der aufgrund eines erhöhten Drucks im Behälter verformbar ist, wenn er in der genannten Umgebung platziert wird, wobei das Verfahren Folgendes umfasst:
Halten jedes der Behälter in einem jeweiligen Behälterhalter (15), so dass verformbare Oberflächen des Behälters durch den Behälterhalter gestützt werden;
Zusammenbauen eines oder mehrerer der Behälter mit ihren Behälterhaltern (15) auf einem Sterilisationsgestell (10), wobei das Sterilisationsgestell (10) Folgendes aufweist:
eine längliche Basis (11) mit einem ersten Ende und einem zweiten Ende an gegenüberliegenden Enden entlang der Basis;
eine Pufferplatte (12), die an einer ersten Position entlang der Basis (11) angeordnet ist; und
eine Kompressionseinheit (13), die eine Druckplatte (20) umfasst, wobei die Kompressionseinheit (13) eine Gewindebohrung umfasst, durch die eine Gewindespindel (21) der Druckplatte (20) eingreifen kann und betätigbar ist, um die Position der Druckplatte in einer Längsrichtung in Bezug auf die Position der Kompressionseinheit (13) einzustellen, wobei sich die Kompressionseinheit an einer zweiten Position in Richtung eines der Enden der Basis (11) befindet;
Einstellen einer Position der Pufferplatte (12) entlang der Basis (11), so dass die Behälter zwischen die Pufferplatte (12) und die Druckplatte (20) passen;
Einstellen einer Position der Druckplatte (20) durch Betätigen der Gewindespindel (21), so dass ein Kontakt zwischen der Druckplatte (20) und jedem aufeinanderfolgenden Behälterhalter (15) bis zur Pufferplatte (12) hergestellt wird.

## Revendications

1. Un râtelier (10) pour tenir un ou plusieurs produits à stériliser dans un environnement à température augmentée, chacun des produits étant contenu dans un conteneur qui est déformable en raison d'une pression accrue à l'intérieur du conteneur lorsqu'il est placé dans ledit environnement, le râtelier (10) comprenant :
une base allongée (11) ayant une première extrémité et une deuxième extrémité à des extrémités opposées le long de la base ;
une plaque tampon (12) située dans une première position le long de la base (11) ;
une unité de compression (13) comprenant une plaque de pression (20), l'unité de compression (13) étant située dans une deuxième position vers l'une des extrémités de la base (11) ; et
un ou plusieurs supports de conteneur (15), situés entre la plaque tampon (12) et la plaque de pression (20), chaque support de conteneur (15) étant adapté pour retenir un conteneur respectif ;
l'unité de compression (13) étant appropriée pour appliquer une pression sur lesdits supports de conteneur (15) ;
**caractérisé en ce que** la plaque tampon (12) est placée sur la base (11), ladite plaque tampon (12) comprenant des moyens de sélection de la première position dans laquelle la plaque tampon (12) est située, parmi une pluralité de premières positions alternatives le long de la base (11) et **en ce que** ;
l'unité de compression (13) comprend un trou fileté adapté pour recevoir une tige filetée (21) de la plaque de pression (20), cette tige filetée pouvant être manipulée pour ajuster la position de la plaque de pression dans une direction longitudinale par rapport à l'emplacement de l'unité de compression (13), de sorte qu'un contact est établi entre la plaque de pression (20) et chaque support de conteneur (15) successif jusqu'à la plaque tampon (12).

2. Le râtelier selon la revendication 1, dans lequel le support de conteneur (15) comprend une attache (22) comprenant deux pattes de maintien (24) pour retenir son conteneur respectif.

3. Le râtelier selon la revendication 2, dans lequel le support de conteneur (15) comprend en outre une plaque de contre-pression (23) sur l'une des pattes de maintien (24) pour retenir son conteneur respectif.

4. Le râtelier selon la revendication 3, dans lequel la plaque de contre-pression (23) comprend une protubérance coopérant avec un profilé (27) de la patte de maintien (24) afin d'ajuster la position de ladite plaque de contre-pression (23) pour amener la plaque de contre-pression à fournir une contre-pression à une partie souhaitée du conteneur.

5. Le râtelier selon l'une quelconque des revendications précédentes, dans lequel la position de l'unité de compression (13) le long de la longueur de la base (11) est réglable.

6. Le râtelier selon l'une quelconque des revendications précédentes, dans lequel la base (11) comprend une rainure longitudinale (16), lesdites pattes de maintien (24) des supports de conteneur (15) étant liées entre elles par une patte de guidage (25), ladite patte de guidage (25) pouvant coulisser le long de la rainure longitudinale (16).

7. Le râtelier selon l'une quelconque des revendications 2 à 6, dans lequel l'une des pattes de maintien (24) des supports de conteneur (15) a un profilé (26) sur ses faces externes et les pattes de maintien restantes (24) des supports de conteneur (15) ont un profilé complémentaire (27) sur leurs faces externes de sorte que les supports de conteneur adjacents s'enclenchent les uns dans les autres lorsqu'ils sont assemblés sur la base (11).

8. Procédé pour la préparation d'un ou plusieurs produits pour la stérilisation dans un environnement à température augmentée, les produits étant chacun contenus dans un conteneur qui est déformable en raison d'une pression accrue à l'intérieur du conteneur lorsqu'il est placé dans ledit environnement, le procédé comprenant les étapes suivantes :
retenir chacun des conteneurs dans un support de conteneur respectif (15) de sorte que des surfaces déformables du conteneur sont maintenues par le support de conteneur ;
assembler un ou plusieurs des conteneurs avec leurs supports de conteneur (15) sur un râtelier de stérilisation (10), le râtelier de stérilisation (10) ayant :
une base allongée (11) ayant une première extrémité et une deuxième extrémité à des extrémités opposées le long de la base ;
une plaque tampon (12) située dans une première position le long de la base (11) ; et
une unité de compression (13) comprenant une plaque de pression (20), l'unité de compression (13) comprenant un trou fileté adapté pour recevoir une tige filetée (21) de la plaque de pression (20), cette tige filetée pouvant être manipulée pour ajuster la position de la plaque de pression dans une direction longitudinale par rapport à l'emplacement de l'unité de compression (13), l'unité de compression étant située dans une deuxième position vers l'une des extrémités de la base (11) ;
ajuster une position de la plaque tampon (12) le long de la base (11) de sorte que lesdits conteneurs s'adaptent entre la plaque tampon (12) et la plaque de pression (20) ;
ajuster une position de la plaque de pression (20) en actionnant ladite tige filetée (21) de sorte qu'un contact est établi entre la plaque de pression (20) et chaque support de conteneur (15) successif jusqu'à la plaque tampon (12).
